# EUROPEAN PATENT SPECIFICATION

(11) **EP 1 227 102 B1**
(45) Date of publication and mention of the grant of the patent: **31.03.2004**
(21) Application number: 02001530.1
(22) Date of filing: 23.01.2002
(51) Int. Cl.: C07H 17/08

(54) **An improved process for the preparation of non-hygroscopic azithromycin dihydrate**
Verbessertes Verfahren zur Herstellung von non-hygroscopischem Azithromycin Dihydrat
Procédé amélioré de préparation de dihydrate d'azithromycine non-hygroscopique

(30) Priority: 29.01.2001 IN BO952001
(43) Date of publication of application: 31.07.2002
(73) Proprietor: Alembic Limited, Vadodara 390003, Gujarat (IN)
(72) Inventor: Rengaraju, Srinivasan, Dr., c/o Alembic Ltd., Vadodara-390003, Gujarat (IN)
(74) Representative: Flaccus, Rolf-Dieter, Dr.

(56) References cited:
- EP-A- 0 298 650
- EP-A- 0 941 999
- EP-A- 1 103 558
- CN-A- 1 093 370
- BAYOD-JASANADA ET AL: 'SYNTHESIS OF 9-DEOXO-9A-AZA-HOMOERYTHROMYCIN A 11,12-HYDROGEN BORATE AND AZITHROMYCIN 11,12-HYDROGEN BORATE. A NEW PROCEDURE TO OBTAIN AZITHROMYCIN DIHYDRATE' J.O.C. vol. 62, no. 21, 1997, EASTON, US, pages 7479 - 7481, XP002061020
- CHEMICAL ABSTRACTS, vol. 124, no. 3, 1996, Columbus, Ohio, US; abstract no. 29525Q, SONG QUANLAI ET AL.: 'Preparation of crystals of azithromycin via crystallization from various solvents' page 29525; column RIGHT;

## Description

The invention relates to an improved process for the production of non-hygroscopic Azithromycin dihydrate.

Azithromycin (1) (USAN generic name for 9-Deoxo-9a-aza-9a-methyl-9a-homo- Eryhromycin A) is a 15 membered ring macrolide belonging to a new class of antibiotics termed as "Azalides", due to the incorporation of nitrogen atom in the macrocyclic ring. It is derived from the 14-membered macrolide .antibiotic Erythromycin A and shows significant improvement in its activity against gram negative organisms compared to Erythromycin A (C J.Dunn and L B Barradell Azithromycin A Review of its Pharmacological properties and use as a 3-day therapy in respiratory tract infections, Drugs, 1996 (March,51 (3)483-505).

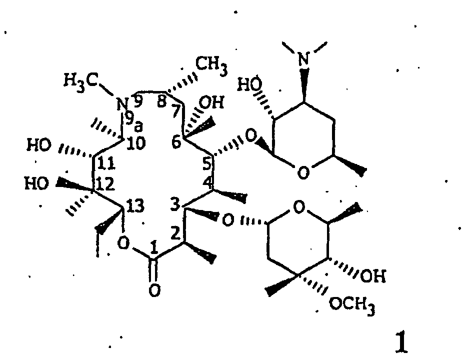

### Background of the invention

Azithromycin was first discovered by G. Kobrehel and S. Djokic (Belgium Patent No. 892357 and its related U.S.Patent No. 4,517,359). S. Djokic et al (J CHEMRESEARCH(S). 1998, 132 and idem miniprint 1998, 1239), have demonstrated the existence of the dihydrate form ofAzithromycin.

In the U.S.Patent 4,517,359, Azithromycin was isolated by evaporation of its chloroform solution under vacuum. A melting point of 113-115°C was reported for this preparation. There is no mention of the crystal form of Azithromycin in the Patent. In all probability, it is an amorphous powder.

Azithromycin preparation was also described by G.M.Bright in U.S. Patent No. 4,474,768 (Corresponding E.P.No.0101186) wherein the amorphous azithromycin foam obtained by evaporation of a methylene chloride solution was crystallized from ethanol : water to give Azithromycin melting at 142°C. In this Patent as well there is no mention of the crystal form.

Azithromycin is known to exist in two crystalline forms. In EP-A-0 298 650 (of Pfizer Inc.; equivalent Indian Patent IN 168896), Allen and Nepveux have shown that Azithromycin crystallized from ethanol : water gives a hygroscopic monohydrate form which melts at 142°C. They have described a method for conversion of the hygroscopic monohydrate form to the dihydrate form of Azithromycin. This involves recrystallization from a mixture of solvents containing tetrahydrofuran and an aliphatic (C₅ - C₇) hydrocarbon in presence of water. The Azithromycin dihydrate thus prepared has a melting point of 126°C. The process of Allen et al suffered from some disadvantages. In this process a mixture of tetrahydrofuran and a hydrocarbon like hexane is used. Mixtures of two organic solvents result in higher recovery costs and besides handling of hydrocarbon solvents requires extra care due to fire hazards. Due to its low flash point and high flammability, hexane causes problems e. g. during centrifugation when the product is to be isolated at commercial scale. Besides, hexane is classified as Class II solvent in ICH guidelines and its presence is not advisable in the final drug preparation. Tetrahydrofuran is hazardous to handle as it can form peroxides in the presence of oxygen. Furthermore, the method of D2 reguires the addition of diatomaceous earth, and azithromycin monohydrate must be dissolved in the solvent, tetrahydrofuran.

S. Djokic et al (Journal of Chemical Research (S) 1998, 132 and idem miniprint, 1998, 1239) also have demonstrated the existence of dihydrate form of Azithromycin.

Bayod Jasanda *et al* in the J. Org Chemistry (1997) 62, 7479-7481, XP-002061020 (which also is disclosed in U.S.Patent 5,869,629) have described a method of preparation of Azithromycin dihydrate by the recrystallization of the hygroscopic form of Azithromycin from acetone : water and agitating the slurry for 24 hrs. The monohydrate is first dissolved in acetone, followed by the addition of water. Again, this method has inherent disadvantages. The conversion of monohydrate to dihydrate form requires stirring for long periods such as for 24 hours.

EP-A-0 941 999 also describes the crystallization of azithromycin first by dissolving azithromycin in aqueous acetone and then precipitating as dihydrate by adjusting pH to alkaline side. According to this document, crude azithromycin is dissolved in water by adjusting the pH 2.0-2.5 with 6N HCl at temp 20-25 °C and then readjusting the pH 9.8 in acetone-water with sodium hydroxide. Adiustment of pH with HCl (6N) is a critical parameter, since it is known that decladinosylation in macrolide is very facile at acidic pH, hence at commercial scale this may cause problems. Re-adjustment of the pH with NaOH leads to the formation of sodium chloride. Hence, if the final product is not properly washed, sulphated ash problem will be encountered with the product. All the reported operations for converting azithromycin crude to azithromycin dihydrate at commercial scales are quite cumbersome. Besides, the time for converting monohydrate to dihydrate in acetone-water is 24 hrs, which is approximately 16-6 hrs more than the present invention which increases the plant occupancy time and requires more man hours, thus making the manufacturing process more expensive.

EP-A-1 103 558 (published after the priority date of the present application) relates to a method for the preparation of Azithromycin dihydrate which is based on re-crystallization from water and tert-butanol.
In the first step of this prior art process, the monohydrate form is dissolved in the organic solvent, e. g. t-butanol. This method requires an additional step where NaOH is added or a step in which the solution is discharged over a mixture of petroleum ether and water. NaOH or water is added after the monohydrate form is dissolved in the organic solvent. Furthermore, this method requires a cooling or lyophilization step.

CN 1093370A discloses a method for obtaining "a short-column azithromycin crystal" by crystallizing the monohydrate form in a mixture of a water-soluble organic solvent and water. This document only describes the production of polymorph forms of azithromycin which are not comparable to or identical with the dihyrdrate crystalline form. The crystals of the Chinese Patent are different from the rhombic crystals of the Azithromycin dihydrate of the present invention. According to the method of CN 1093370A, the monohydrate is dissolved in a hot mixture of organic solvent (preferably acetone) and water. Crystallization is induced or promoted by gradually adding water. This method is disadvantageous in that it requires an additional step of dissolving the monohydrate in a hot mixture of organic solvent and water, and it requires very high quantities of solvent. Furthermore, the polymorph formation is critically dependent on a variety of process parameters.

The monohydrate form of Azithromycin is difficult to handle during its formulation into capsules or other forms due to its hygroscopicity. Hence the stable dihydrate form is used in the formulations of Azithromycin. Due to this importance of Azithromycin dihydrate in formulations of azithromycin, methods of conversion of unstable monohydrate form to stable dihydrate form is required.

It is therefore an objective of the invention to provide an improved process for the production of non-hygroscopic Azithromycin dihydrate.

It is a further object of the present invention to provide a Azithromycin dihydrate employing selected solvents whereby the process can be carried out at room temprature with no additional energy input.

It is a further objective of the invention to provide a process for the production of Azithromycin dihydrate wherein the process is quick and formation of crystals can be easily observed.

These are other objects of the invention that will now be described in the embodiments of the specification.

### Summary of the Invention

The invention discloses an improved process for preparing non-hygroscopic Azithromycin dihydrate which comprises
- preparation of a suspension of Azithromycin monohydrate, water and a solvent selected from the group consisting of dimethylformamide, dimethylacetamide, acetonitrile and iso-propanol,
- stirring the suspension to form a slurry,
- filtering the slurry, and
- drying the slurry under a vacuum to yield crystals of non-hygroscopic Azithromycin dihydrate.

The crystals of Azithromycin dihydrate are subjected to filtration and drying in any conventional manner.

### Description of the invention

The monohydrate form of Azithromycin is difficult to handle during its formulation into capsules or other forms due to its hygroscopicity. Hence the stable dihydrate form is used in the formulations of Azithromycin. Due to this importance of Azithromycin dihydrate in formulations of azithromycin, methods of conversion of unstable monohydrate form to stable dihydrate form is desired.

The present invention discloses a simple and novel method for the conversion of hygroscopic monohydrate form of azithromycin to the dihydrate form of azithromycin. The inventors have found due to the experimentation done by them that the hygroscopic monohydrate of azithromycin can be converted to the stable dihydrate form by agitating a slurry of monohydrate form in a water solvent mixture.

The solvent which is employed in the process of the invention is selected from the group consisting of (1) dimethyl formamide (2) dimethyl acetamide (3) acetonitrile (4) *iso*-propanol. The agitation of the slurry is carried out at . ambient temperature without the requirement of heating or cooling the mixture. The transformation of the azithromycin monohydrate crystal' to azithromycin dihydrate crystal can be easily followed by observing the crystal slurry under the microscope, as the crystal habit of both the forms are different.

The monohydrate crystals are of cubic habit which during the agitation in aqueous solvent mixture slowly gets converted to the dihydrate form. The crystals of dihydrate are of rhombic habit which are easily distinguishable under the microscope from the cubic habit of the monohydrate, thus making the process amenable for quick and easy process control. When all the crystals are of rhomboid type the agitation is stopped and the slurry is filtered and dried under vacuum. The stirring is usually carried out for 2-18 hrs, by which time the transformation of the monohydrate to dihydrate takes place. Preferably the ideal water to solvent ratio is 1:1 and the slurry concentration is kept at 50%, so as to ensure maximum recovery of the dihydrate. Lower concentrations of water in the solvent is not contraindicated for the conversion but it is avoided as loss by solubility of Azithromycin in such systems will increase.

The agitation of the slurry of Azithromycin monohydrate in solvent : water mixture can be carried out by the conventional methods of agitation such as magnetic stirring in the laboratory scale or mechanical agitation as practiced in industrial scale.

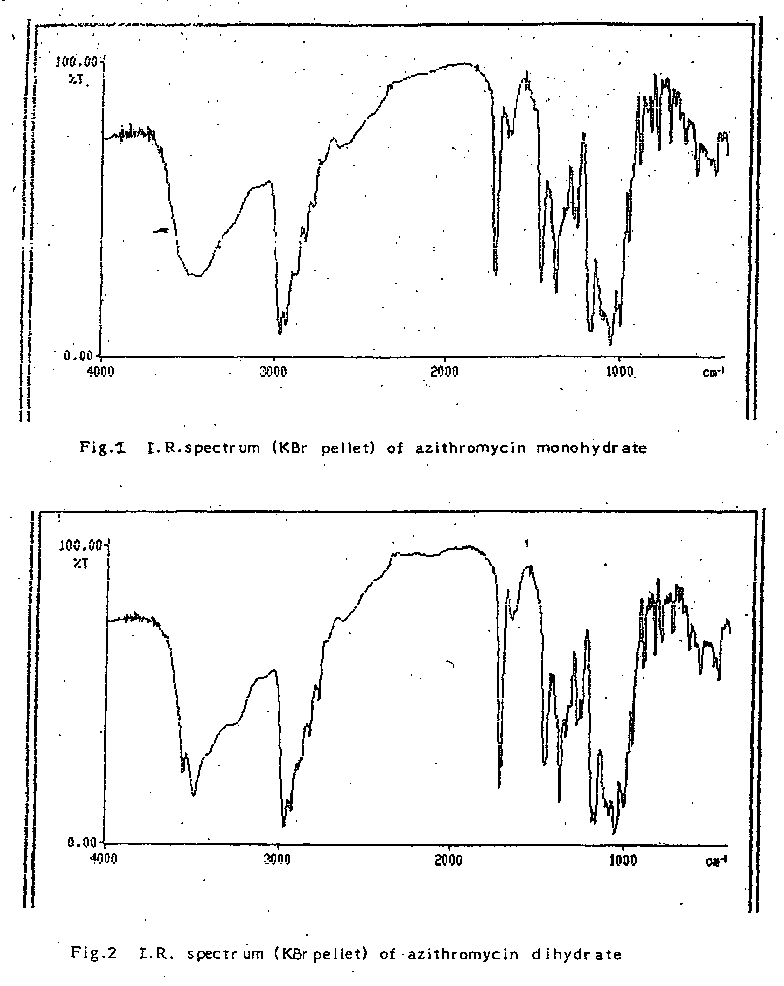

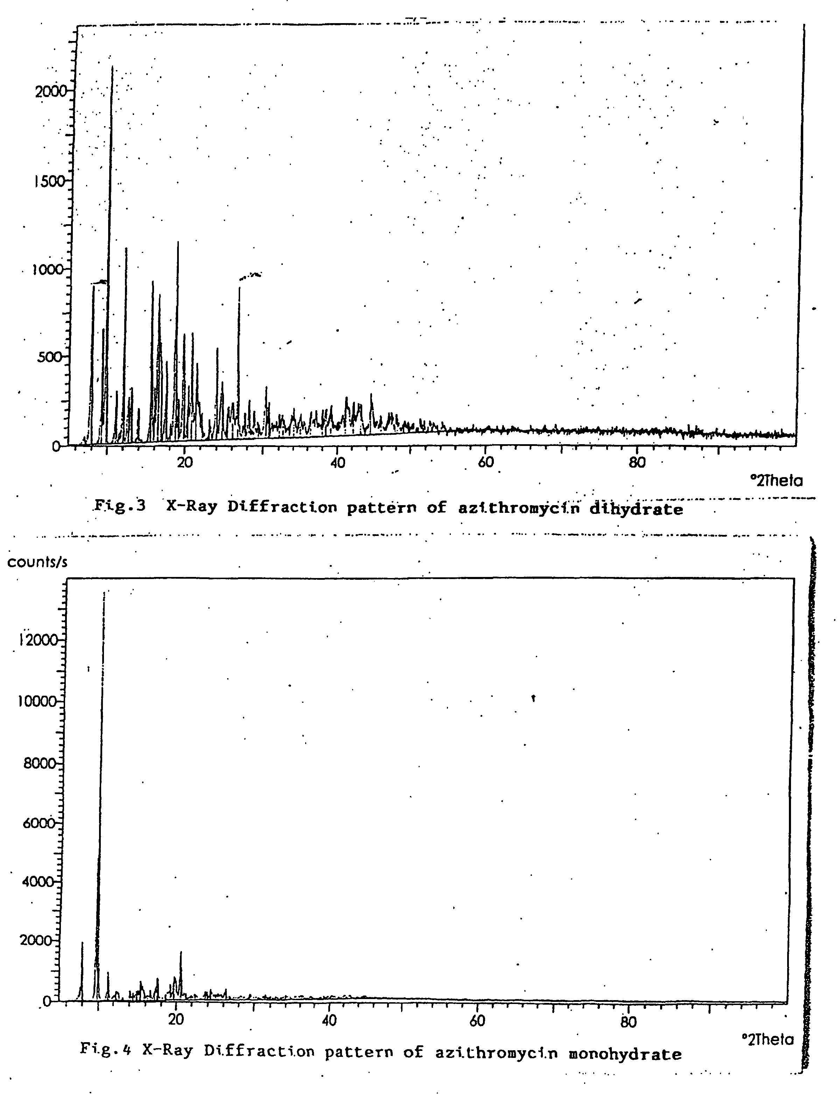

The Azithromycin dihydrate is easily distinguished from the monohydrate by their characteristic solid state (KBr, pellet) IR spectra (Fig 1 & 2). The monohydrate shows a broad peak in the hydroxyl stretching region at 3450' cm⁻¹ (broad), whereas the dihydrate shows two peaks in this region at 3560 cm⁻¹ (shoulder) and 3495 cm⁻¹. There are also characteristic absorption for the two forms in the C-0, C-N stretching regions (1000-1200 cm⁻¹) (Fig 1 & 2). The two forms are also distinguished by their characteristic x-ray diffraction patterns (Fig 3 & 4).

Unlike any of the prior Patents or methods the invention provides the choice of using any of the four solvents - dimethyl formamide, dimethyl acetamide, acetonitrile or *iso*-propanol - in the process. As the process is carried out at ambient temperature, no additional energy input is needed. Besides, the process can be easily and quickly followed by observing the crystal habit under a microscope it is possible to terminate the agitation (stirring) at optimum time. Azithromycin is produced by the reductive methylation of 9-Deoxo -9-α-aza-9a-homoerythromycin using formaldehyde-formic acid mixture. The reaction generates certain impurities which can be removed in the aqueous-solvent slurrying step of the monohydrate to dihydrate conversion.

The invention will now, be described with reference to the following examples which are only illustrative and should in no way be understood to limit the scope of the invention in any manner whatsoever:

### Preparation of hygroscopic Azithromycin monohydrate

9-Deoxo-9a-aza-9a-homoerythromycin A (73.5g - 0.1 mole) was dissolved in 250ml acetone. To this solution formic acid (19ml) followed by formaldehyde (37%, 20ml, ) were added and refluxed for 24 hrs. The pH of the reaction mixture was adjusted with alkali to 10.5 and filtered to remove particles. To the filtered acetone solution equal volume of water was added to precipitate azithromycin hygroscopic monohydrate as cube shaped crystals. The crystals were filtered and dried under vacuum at 50° C to give 65g of azithromycin monohydrate melting at 130-131°C having a water content of 3.42% (by Karl Fischer titration method). This sample of hygroscopic monohydrate has a characteristic solid state (KBr pellet) IR spectrum (Fig 1) and a characteristic x-ray diffraction pattern (Fig 4). The crystals absorbed moisture on exposure to ambient atmosphere and a moisture content of 5.4% was reached in 48 hrs.

### Example 1 : Preparation of Azithromycin dihydrate from hygroscopic Azithromycin monohydrate using iso-propanol : water mixture.

10gms of hygroscopic azithromycin-monohydrate was suspended in a mixture of iso-propanol (10ml) and water (10ml) and stirred at ambient temperature. The transformation of cubical crystals of monohydrate form to the rhomboid : form crystals of dihydrate was followed by checking the crystal habit under a microscope at every two hour interval. At 16 hours the rhomboid dihydrate 'crystals only were seen. The slurry was filtered and dried under vacuum at 50°C to give 9.8g of azithromycin dihydrate. It had a melting point of 126-128°C and water content of 4.65% (Theoretical 4.586) (by Karl-Fischer titration method). It has a characteristic solid state IR spectrum (KBr pellet) (Fig 2) and x-ray diffraction pattern (Fig 3). On exposure to ambient atmosphere there was no change in the moisture content of the dihydrate crystals.

### Example 2 : Preparation of Azithromycin dihydrate from hygroscopic Azithromycin monohydrate using acetonitrile : water mixture.

10gms of hygroscopic azithromycin-monohydrate was suspended in a mixture of acetonitrile (10ml) and water (10ml) and stirred at ambient temperature. The transformation of cubical crystals of monohydrate form to the rhomboid form crystals of dihydrate was followed by checking the crystal habit under a microscope at every two hour interval. At 8 hours the rhomboid dihydrate crystals only were seen. The slurry was filtered and dried under vacuum at 50°C to give 9.8g of azithromycin dihydrate. It had a melting point of 126-128°C and water content of 4.68% (Theoretical 4.586) (by Karl-Fischer titration method). It has a characteristic solid state IR spectrum (KBr pellet) (Fig 2) and x-ray diffraction pattern (Fig 3). On exposure to ambient atmosphere there was no change in the moisture content of the dihydrate crystals.

### Example 3 : Preparation of Azithromycin dihydrate from hygroscopic Azithromycin monohydrate using dimethyl formamide : water mixture.

10gms of hygroscopic azithromycin-monohydrate was suspended in a mixture of dimethyl formamide (10ml) and water (10ml) and stirred at ambient temperature. The transformation of cubical crystals of monohydrate form to the rhomboid form crystals of dihydrate was followed by checking the crystal habit under a microscope at hourly interval. At 3 hours the rhomboid dihydrate crystals only were seen. The slurry was filtered and dried under vacuum at 50°C to give 9.8g of azithromycin dihydrate. It had a melting point of 126-128°C and water content of 4.6% (Theoretical 4.586) (by Karl-Fischer titration method). It has a characteristic solid state IR spectrum (KBr pellet) (Fig 2) and x-ray diffraction pattern (Fig 3). On exposure to ambient atmosphere there was no change in the moisture content of the dihydrate crystals.

### Example 4 : Preparation of Azithromycin dihydrate from hygroscopic Azithromycin monohydrate using dimethyl acetamide: water mixture.

10gms of hygroscopic azithromycin-monohydrate was suspended in a mixture of dimethyl acetamide (10ml) and water (10ml) and stirred at ambient temperature. The transformation of cubical crystals of monohydrate form to the rhomboid form crystals of dihydrate was followed by checking the crystal habit under a microscope at every two hour interval. At 4 hours the rhomboid dihydrate crystals only were seen. The slurry was filtered and dried under vacuum at 50°C to give 9.8g of azithromycin dihydrate. It had a melting point of 126-128°C and water content of 4.63% (Theoretical 4.586) (by Karl-Fischer titration method). It has a characteristic solid state IR spectrum (KBr pellet) (Fig 2) and x-ray diffraction pattern (Fig 3). On exposure to ambient atmosphere there was no change in the moisture content of the dihydrate crystals.

## Claims

1. A process for the preparation of non-hygroscopic Azithromycin dihydrate which comprises
- preparation of a suspension of Azithromycin monohydrate, water and a solvent selected from the group consisting of dimethylformamide, dimethylacetamide, acetonitrile and iso-propanol,
- stirring the suspension to form a slurry,
- filtering the slurry, and
- drying the slurry under a vacuum to yield crystals of non-hygroscopic Azithromycin dihydrate.

2. A process as claimed in claim 1, wherein the water and the solvent are in the ratio of 1:1.

3. A process as claimed in claim 1, wherein the stirring of the suspension is carried out for about 2 to 18 hours.

4. A process as claimed in claim 1, wherein the slurry is monitored for a crystal habit of the Azithromycin monohydrate and the Azithromycin dihydrate such that stirring of the slurry is halted when the cubic habit of the Azithromycin monohydrate crystals has converted into a rhombic crystal habit characteristic of the Azithromycin dihydrate,

## Patentansprüche

1. Verfahren zur Herstellung von nicht-hygroskopischem Azithromycindihydrat, das umfasst
- Herstellung einer Suspension von Azithromycinmonohydrat, Wasser und einem Lösungsmittel, das aus der Gruppe gewählt ist, die aus Dimethylformamid, Dimethylacetamid, Acetonitril und Isopropanol besteht,
- Rühren der Suspension, um eine Aufschlämmung (Slurry) herzustellen,
- Filtrieren der Aufschlämmung, und
- Trocknen der Aufschlämmung im Vakuum, um Kristalle von nicht-hygroskopischem Azithromycindihydrat zu erhalten.

2. Verfahren nach Anspruch 1, bei dem Wasser und Lösungsmittel im Verhältnis 1 : 1 vorliegen.

3. Verfahren nach Anspruch 1, bei dem die Suspension etwa 2 bis 18 Stunden gerührt wird.

4. Verfahren nach Anspruch 1, bei dem die Aufschlämmung auf einen Kristallhabitus des Azithromycinmonohydrats und des Azithromycindihydrats hin überwacht wird in der Weise, dass das Rühren der Aufschlämmung gestoppt wird, wenn der kubische Habitus der Azithromycinmonohydratkristalle sich in einen rhombischen Kristallhabitus verwandelt hat, der charakteristisch ist für Azithromycindihydrat.

## Revendications

1. Procédé de préparation de dihydrate d'azithromycine non hygroscopique qui comprend :
- la préparation d'une suspension de monohydrate d'azithromycine, d'eau et d'un solvant choisi dans le groupe comprenant le diméthylformamide, le diméthylacétamide, l'acétonitrile et l'iso-propanol,
- l'agitation de la suspension pour former une suspension épaisse,
- la filtration de la suspension épaisse, et
- le séchage de la suspension épaisse sous vide pour obtenir des cristaux de dihydrate d'azithromycine non hygroscopique.

2. Procédé selon la revendication 1, dans lequel l'eau et le solvant sont dans le rapport 1:1.

3. Procédé selon la revendication 1, dans lequel l'agitation de la suspension est effectuée pendant environ 2 à 18 heures.

4. Procédé selon la revendication 1, dans lequel la suspension épaisse est contrôlée quant à la forme cristalline du monohydrate d'azithromycine et du dihydrate d'azithromycine de telle façon que l'agitation de la suspension épaisse est stoppée lorsque la forme cubique des cristaux de monohydrate d'azithromycine a été convertie en une forme cristalline rhombique, caractéristique du dihydrate d'azithromycine.
